# EUROPÄISCHE PATENTSCHRIFT

(11) **EP 3 363 492 B1**
(45) Veröffentlichungstag und Bekanntmachung des Hinweises auf die Patenterteilung: **21.10.2020**
(21) Anmeldenummer: 17156740.7
(22) Anmeldetag: 17.02.2017
(51) Int. Cl.: A61M 37/00, A01K 11/00

(54) **NADELMODUL FÜR EINE ANTRIEBSGESTEUERTE VORRICHTUNG ZUM WIEDERHOLTEN AUFSTECHEN EINER MENSCHLICHEN ODER EINER TIERISCHEN HAUT UND HANDGERÄT**
NEEDLE MODULE FOR A DRIVE-MANAGED DEVICE FOR REPEATED PUNCTURING OF HUMAN OR ANIMAL SKIN AND HAND-HELD DEVICE
MODULE D'AIGUILLES POUR UN DISPOSITIF COMMANDÉ PAR ENTRAÎNEMENT DESTINÉ AU PERÇAGE RÉPÉTÉ D'UNE PEAU HUMAINE OU ANIMALE ET APPAREIL MANUEL

(43) Veröffentlichungstag der Anmeldung: 22.08.2018
(73) Patentinhaber: MT.DERM GmbH, 14167 Berlin (DE)
(72) Erfinder: Neumetzler, Heiko, 12623 Berlin (DE)
(74) Vertreter: Bittner, Thomas L.

(56) Entgegenhaltungen:
- EP-A1- 2 796 164
- DE-A1-102014 012 896
- DE-U1-202010 013 095
- TW-A- 201 406 322
- US-B2- 8 029 527

## Beschreibung

Die Erfindung betrifft ein Nadelmodul für eine antriebsgesteuerte Vorrichtung zum wiederholten Aufstechen einer menschlichen oder einer tierischen Haut sowie ein Handgerät.

### Hintergrund

Derartige Vorrichtungen werden genutzt, um die Haut lokal aufzustechen, wodurch der Eintrag einer Substanz in die Haut ermöglicht ist. Beispielsweise kann es sich hierbei um einen Farbstoff zum Herstellen eines Tattoos oder von permanentem Make-up handeln. Aber auch der Eintrag einer medizinischen oder einer kosmetischen Substanz über die Haut kann so ermöglicht werden

Bekannt sind Handgeräte mit einem Modulaufbau. So weisen bekannte Handgeräte ein Antriebsmodul sowie ein hieran koppelndes Nadelmodul auf, wobei die Module lösbar verbunden sein können. In einem Gehäuse des Antriebsmoduls ist eine Antriebseinrichtung vorgesehen, beispielsweise ein Antriebsmotor. Mit der Antriebseinrichtung wird eine Antriebskraft mit einer Wiederholfrequenz bereitgestellt. Das an das Antriebsmodul koppelnde Nadelmodul weist eine Stecheinrichtung auf, die eine Einzelnadel oder eine Nadelgruppe aufweisen kann. Die Stecheinrichtung ist an einer Nadelaufnahme angeordnet, die in dem Gehäuse des Nadelmoduls in Längsrichtung vor und zurück verlagerbar ist. Mithilfe eines Kopplungsmechanismus wird die von der Antriebseinrichtung bereitgestellte Antriebskraft von der Antriebseinrichtung auf die Nadelaufnahme übertragen, sodass die Nadelaufnahme mit der Stecheinrichtung im Betrieb vor- und zurückbewegt wird, wodurch eine Stechspitze der Stecheinrichtung zumindest in einer distalen Stellung außerhalb des Gehäuses des Nadelmoduls angeordnet ist, so dass die Haut lokal aufgestochen werden kann.

Im Dokument DE 10 2014 012 896 A1 ist ein Adapter mit Amplitudenuntersetzung zum repetitiven Einstechen von Stoffen in organische Haut offenbart. Der Adapter umfasst ein erstes Masse-Federsystem, bestehend aus einem antriebsseitigen austauschbaren Koppelmechanismus mit Antriebsstange, die in den Axiallagern des Gehäuses gleitend geführt ist, und einem Federelement, und ein zweites Masse-Federsystem, bestehend aus den Federelementen und, dem Federnblock, der Abtriebsstange, die in den abtriebsseitigen Axiallagern des Gehäuses gleitend geführt ist, und einem abtriebsseitigen austauschbaren Koppelmechanismus. Beide Federsysteme sind derart konfiguriert, dass eine in die Antriebsseite mit einem Hub X eingebrachte repetitive Bewegung verlustarm in einen kleineren Hub Y auf der Abtriebsseite umgesetzt wird.

Das Dokument DE 20 2010 013 095 U1 beschreibt eine Vorrichtung zum wiederholten Aufstechen von Haut, mit einem Gehäuse, einer Antriebseinrichtung, die in dem Gehäuse aufgenommen und konfiguriert ist, eine Antriebskraft bereitzustellen, einem Kopplungsmechanismus, der in dem Gehäuse aufgenommen ist, und einer Stecheinrichtung, die über den Kopplungsmechanismus an die Antriebseinrichtung koppelt, derart, dass mittels des Kopplungsmechanismus die Antriebskraft für eine repetierende Stechbewegung auf die Stecheinrichtung gekoppelt wird. Die Stecheinrichtung weist eine Basis mit einer hieran gebildeten distalen Wirkfläche auf, über die mehrere beabstandete Stechelemente verteilt sind. Die Basis mit der hieran gebildeten distalen Wirkfläche ist relativ zum Gehäuse verkippbar gelagert.

### Zusammenfassung

Aufgabe der Erfindung ist es, ein Nadelmodul für eine antriebsgesteuerte Vorrichtung zum wiederholten Aufstechen einer menschlichen oder einer tierischen Haut sowie ein Handgerät mit verbesserten Betriebseigenschaften anzugeben.

Die Aufgabe wird gelöst durch ein Nadelmodul für eine antriebsgesteuerte Vorrichtung zum wiederholten Aufstechen einer menschlichen oder einer tierischen Haut sowie ein Handgerät nach den Ansprüchen 1 und 15. Alternative Ausgestaltungen sind Gegenstand von abhängigen Unteransprüchen.

Nach einem Aspekt ist ein Nadelmodul für eine antriebsgesteuerte Vorrichtung zum wiederholten Aufstechen einer menschlichen oder einer tierischen Haut geschaffen. Das Nadelmodul weist ein Gehäuse auf, in welchem ein Nadelmodul in Längsrichtung des Gehäuses vor und zurück verlagerbar angeordnet ist. An der Nadelaufnahme ist eine Stecheinrichtung angeordnet, derart, dass die Stecheinrichtung aufgrund der Bewegung der Nadelaufnahme zwischen einer proximalen und einer distalen Stellung, in welcher eine Stechspitze der Stecheinrichtung außerhalb des Gehäuses angeordnet ist, verlagerbar ist. Es ist ein Koppelbauteil vorgesehen, welches an die Nadelaufnahme koppelt, derart, dass eine von einer an das Koppelbauteil koppelbaren Antriebseinrichtung mit einer Wiederholfrequenz bereitgestellte Antriebsbewegung auf die Nadelaufnahme einleitbar ist. Zwischen der Nadelaufnahme und dem Koppelbauteil ist ein Dämpfungsbauteil angeordnet, welches eine Kraftübertragung zwischen der Nadelaufnahme und dem Koppelbauteil dämpfend ausführt.

Nach einem weiteren Aspekt ist ein Handgerät zum wiederholten Aufstechen einer menschlichen oder einer tierischen Haut geschaffen, welches ein Antriebsmodul mit einer Antriebseinrichtung aufweist, mit der eine Antriebsbewegung mit einer Wiederholfrequenz bereitgestellt ist. Ein Nadelmodul ist mit dem Antriebsmodul verbunden, derart, dass die Antriebsbewegung auf die Nadelaufnahme mit der Stecheinrichtung gekoppelt ist.

Die Stecheinrichtung kann mit einer Nadel oder einer Nadelgruppe gebildet sein.

Die Antriebseinrichtung stellt eine nicht manuell, sondern maschinell oder automatisiert erzeugte Antriebsbewegung zur Verfügung. Hierbei kann die Antriebseinrichtung beispielsweise einen elektrischen Motor aufweisen.

Die Wiederholfrequenz, mit der die Antriebsbewegung bereitgestellt ist, kann zwischen etwa 40 und etwa 200 Hz betragen.

Das Nadelmodul kann als Einwegmodul ausgeführt sein. Bei dem Nadelmodul können ein oder mehrere Bauteile aus einem Kunststoffmaterial hergestellt sein, beispielsweise mittels Spritzgießen.

Das Koppelbauteil kann ein- oder mehrstückig ausgeführt sein. Ähnlich kann das Gehäuse des Nadelmoduls ein- oder mehrstückig ausgebildet sein, wobei im Fall der Mehrstückigkeit Gehäusekomponenten lösbar miteinander verbunden sein können.

Das Dämpfungsbauteil kann mit Koppelbauteil und / oder der Nadelaufnahme über eine formschlüssige Verbindung verbunden sein.

An dem Gehäuse des Nadelmoduls kann eine Öffnung vorgesehen sein, um in das Gehäuseinnere eine Substanz einzubringen, welche auf die Haut angewendet werden soll. Hierbei kann die Substanz in einem Gehäuseinnenraum eingebracht werden, der mit einer vorderseitigen Modulöffnung in Verbindung steht, die auch als Nadelöffnung bezeichnet werden kann und durch welche hindurch sich die Stecheinrichtung erstreckt, zumindest in der distalen Stellung.

Die distale Stellung entspricht einer ganz oder teilweise aus- oder vorgefahrenen Stellung der Nadelaufnahme mit der Stecheinrichtung. Die Stechspitze der Stecheinrichtung ist dann außerhalb des Gehäuses angeordnet, sodass die Haut lokal aufgestochen werden kann. Hierbei erstreckt sich die Stecheinrichtung durch eine vorderseitige Öffnung des Gehäuses des Nadelmoduls hindurch, die auch als Nadelöffnung bezeichnet werden kann. Mittels Zurückbewegen aus der distalen Stellung werden die Nadelaufnahme sowie die hieran angeordnete Stecheinrichtung in eine eingefahrene Stellung verlagert, die als proximale Stellung bezeichnet werden kann und in welcher die Stechspitze im Vergleich zur distalen Stellung in Richtung Nadelmodul zurückversetzt ist, wobei die Stechspitze innerhalb oder außerhalb des Gehäuses angeordnet sein kann, beispielsweise innerhalb des Gehäuses hinter der vorderseitigen Öffnung.

Das Dämpfungsbauteil kann aus einem elastisch nachgebenden Material sein. Ein elastisches Kunststoffmaterial kann zum Einsatz kommen. Bei dieser oder anderen Ausführungsformen kann das Dämpfungsbauteil ein- oder mehrstückig ausgeführt sein. Das Dämpfungsbauteil kann in den verschiedenen Ausgestaltungen im Querschnitt zum Beispiel rund oder eckig ausgebildet sein. In einer Ausführungsform weist das Dämpfungsbauteil eine zylindrische Form auf.

Gegenüberliegende Endabschnitte des Dämpfungsbauteils können jeweils direkt an die Nadelaufnahme einerseits und das Koppelbauteil andererseits koppeln. Die gegenüberliegenden Endabschnitte des Dämpfungsbauteils können jeweils im Berührungskontakt mit der Nadelaufnahme und dem Koppelbauteil stehen.

Das Dämpfungsbauteil kann an der Nadelaufnahme und / oder dem Koppelbauteil gegen ein relatives Verrutschen, insbesondere quer zur Längsrichtung des Gehäuses, gesichert sein. Zu diesem Zweck kann das Dämpfungsbauteil an der Nadelaufnahme und / oder im Koppelbauteil in einer zugeordneten Aufnahme angeordnet sein, beispielsweise einer Vertiefung an der Nadelaufnahme und / oder dem Koppelbauteil. Alternativ oder ergänzend kann an einem dem Dämpfungsbauteil zugewandten Ende der Nadelaufnahme und / oder des Koppelbauteils ein Vorsprung gebildet sein, der in eine zugeordnete Vertiefung am Dämpfungsbauteil eingreift.

Eine vom Dämpfungsbauteil bereitgestellte Dämpfung kann mittels einer Einstelleinrichtung einstellbar sein. Mittels der Einstelleinrichtung können ein Härtegrad der Dämpfung und / oder eine Länge eines Dämpfungswegs eingestellt werden, zum Beispiel das Ausmaß einer Stauchung oder eines Zusammendrückens des Dämpfungselements bei endseitiger Druckbelastung.

Die Einstelleinrichtung kann ein auf einer Innenwand des Gehäuses, an der Nadelaufnahme und / oder dem Koppelbauteil verlagerbar angeordnetes Einstellbauteil aufweisen. Das Einstellbauteil kann zum Beispiel eine Bewegung der Nadelaufnahme und / oder des Koppelbauteils begrenzen, zum Beispiel dadurch, dass mit dem Einstellbauteil ein Anschlag bereitgestellt ist, welcher verlagerbar ist, um so die Dämpfung einzustellen. Alternativ oder ergänzend kann das Einstellbauteil eine elastische Verformung des Dämpfungsbauteils begrenzend oder einschränkend ausgeführt sein.

Die Einstelleinrichtung kann eingerichtet sein, einen Dämpfungsweg einzustellen. Der Dämpfungsweg kann das Ausmaß bestimmen, in welchem Umfang das Dämpfungsbauteil stauchbar oder zusammendrückbar ist im Fall der Dämpfung. Mittels Verlagern des Einstellbauteils kann der Dämpfungsweg verändert werden. Hierbei kann beispielsweise das Einstellbauteil einen Anschlag bereitstellen, dessen Lage relativ zur Nadelaufnahme und / oder zum Koppelbauteil einstellbar ist.

Alternativ oder ergänzend kann die Einstelleinrichtung eingerichtet sein, eine elastische Verformung, zum Beispiel eine Ausbauchung infolge einer elastischen Verformung, zu begrenzen, wobei der Grad der Begrenzung einstellbar sein kann.

Die Einstelleinrichtung kann von außerhalb des Gehäuses bedienbar sein, um die Dämpfung einzustellen. Es kann ein Stellbauteil vorgesehen sein, welches zum Bedienen von außen durch eine Substanzöffnung des Gehäuses zugänglich ist. An dem Stellbauteil kann zum Beispiel mit einem Schraubendreher angesetzt werden, um das Einstellelement zu verlagern. Die Verlagerung kann hierbei oder in anderen Ausführungen in Längsrichtung der Nadelaufnahme erfolgen. Die Substanzöffnung kann eine Farbstofföffnung sein, die eingerichtet ist, hierdurch einen Farbstoff ins Gehäuse einzubringen, der auf die Haut appliziert werden soll. Auch andere Substanzen können mittels der Vorrichtung appliziert werden, zum Beispiel kosmetische oder medizinische Stoffe.

In einer Ausführung kann alternativ oder ergänzend ein von außerhalb des Gehäuses zu betätigender Einstellmechanismus vorgesehen sein. Hierbei kann zum Beispiel das Einstellbauteil oder -element an ein von außen zugängliches Stellbauteil und ein im Gehäuse aufgenommenes weiteres Stellbauteil mit einem Gewinde koppeln, derart, dass beim Drehen des Stellbauteils das Einstellbauteil mitgenommen und aufgrund seiner Kopplung an das weitere Stellbauteil mit dem Gewinde in Längsrichtung der Nadelaufnahme verlagert wird, wodurch eine Überlappung mit dem Dämpfungsbauteil eingestellt wird. Das Dämpfungsbauteil kann rückseitig an das weitere Stellbauteil koppeln, welches vorderseitig an die Nadelaufnahme koppelt. Das Stellbauteil kann von außen durch eine Gehäuseöffnung hindurch betätigbar sein, insbesondere drehbar. Es kann alternativ vorgesehen sein, dass das weitere Stellbauteil mit dem Gewinde einstückig mit der Nadelaufnahme gebildet ist, so dass in diesem Fall das Gewinde an der Nadelaufnahme angeordnet ist, an welche rückseitig das Dämpfungsbauteil koppelt.

Die Nadelaufnahme und das Koppelbauteil können über eine zugeordnete und eine Längsverkürzung aufgrund der Dämpfung des Dämpfungsbauteils zulassende Bauteilverlängerung miteinander verbunden sein. Bei dieser Ausgestaltung können Nadelaufnahme und Koppelbauteil trotz des hierzwischen angeordneten Dämpfungsbauteils in direktem Kontakt stehen. Zum Beispiel kann eine Teleskopverlängerung vorgesehen sein, die sich verkürzt, wenn auf das Dämpfungsbauteil Druckkraft ausgeübt wird, insbesondere dann, wenn die Stechspitze der Stecheinrichtung beim Ausfahren auf den Widerstand der Haut trifft. Bei der Bauteilverlängerung können sich ineinanderschiebende Abschnitte vorgesehen sein. Abschnitte der Bauteilverlängerung kann an der Nadelaufnahme und / oder an dem Koppelbauteil gebildet sein.

Die Bauteilverlängerung kann sich längs des Dämpfungsbauteils innerhalb und / oder außerhalb desselben erstrecken. Die Bauteilverlängerung kann sich in einer Ausgestaltung durch eine Öffnung des Dämpfungsbauteils durch dieses hindurch erstrecken. Alternativ oder ergänzend kann die Bauteilverlängerung das Dämpfungsbauteil außen zumindest teilweise umfassend ausgeführt sein.

In dem Gehäuse können die Nadelaufnahme und das Dämpfungsbauteil in einem stechseitigen Gehäuseraum und das Kopplungsbauteil in einem antriebsseitigen Gehäuseraum angeordnet sein, wobei der stechseitige und der antriebsseitige Gehäuseraum mittels einer Membran getrennt sind. Die Membran, die bevorzugt aus einem elastisch nachgebenden Material ist, ist an dem Gehäuse aufgenommen und kann die Gehäuseräume gegeneinander abdichten. In einer Ausgestaltung kann vorgesehen sein, dass sich die Membran von einem antriebsseitigen Ende des Gehäuses her in das Gehäuseinnere erstreckt. Die Membran kann als geschlossene Membran ausgeführt sein. Alternativ kann die Membran eine gedichtete Öffnung aufweisen, um durch diese hindurch die Kopplung zwischen Nadelmodul und Koppelbauteil zu ermöglichen. Von der Membranöffnung ausgehend kann sich rückseitig zur Antriebsseite hin das Koppelbauteil erstrecken, welches seinerseits bei dieser oder anderen Ausführungsformen noch vollständig oder nur zum Teil innerhalb des Gehäuses angeordnet ist. In einer Ausführung kann ein antriebsseitiges Ende des Koppelbauteils in einer Ausgangsstellung, welche der proximalen Stellung der Nadelaufnahme entspricht, rückseitig aus dem Gehäuse des Nadelmoduls vorstehen. Ein antriebsseitiges Ende des Dämpfungsbauteils kann in die Membranöffnung eingesteckt sein. Hierbei kann vorgesehen sein, dass sich das antriebsseitige Ende des Dämpfungsbauteils dann auch in eine zugeordnete Aufnahme am stechseitigen Ende des Koppelbauteils erstreckt, welches der Nadelaufnahme mit der Stecheinrichtung zugewandt ist.

Das Dämpfungsbauteil kann einstückig mit der Membran ausgeführt sein. Eine solche einstückige Anformung an der Membran kann beispielsweise mittels Spritzgießen realisiert sein, zum Beispiel 2K-Spritzen. Mit der Anformung kann ein Hülsenabschnitt gebildet sein, durch welchen hindurch die Kopplung zwischen Nadelaufnahme und Koppelbauteil gebildet sein kann. Der vordere Bereich des Dämpfungsbauteils kann eine Öffnung aufweisen, in die die Stecheinrichtung eingesetzt ist. Hierdurch kann die bei alternativen Ausführungen vorgesehene Nadelaufnahme entfallen. Diese Funktion wird von dem Dämpfungsbauteil übernommen.

Das Dämpfungsbauteil kann als eine vorderseitige Verlängerung der Membran gebildet sein.

Ein stechseitiges Ende des Koppelbauteils kann an einer Membranöffnung angeordnet sein.

Die Membran kann sich in Längsrichtung des Gehäuses und das Koppelbauteil umgreifend erstrecken. Die Membran kann hierbei mit einer Topfform ausgebildet sein.

Das Dämpfungsbauteil kann einstückig mit der Nadelaufnahme ausgeführt sein. Hierbei kann die Nadelaufnahme aus einem elastischen Material bestehen, zum Beispiel einem Elastomer.

Die vorangehend in Verbindung mit dem Nadelmodul erläuterten Ausgestaltungen können entsprechend bei dem Handgerät zum wiederholten Aufstechen einer menschlichen oder einer tierischen Haut vorgesehen sein.

### Beschreibung von Ausführungsbeispielen

Nachfolgend werden weitere Ausführungsbeispiele unter Bezugnahme auf Figuren einer Zeichnung näher erläutert. Hierbei zeigen:
- Fig. 1: eine schematische Darstellung eines Handgeräts zum wiederholten Aufstechen einer menschlichen oder einer tierischen Haut mit einem Nadelmodul und einem Antriebsmodul;
- Fig. 2: eine perspektivische Darstellung eines Nadelmoduls im Schnitt;
- Fig. 3: eine perspektivische Darstellung eines Teils des Nadelmoduls aus Fig. 2;
- Fig. 4: eine weitere schematische Darstellung des Nadelmoduls aus Fig. 1 im Schnitt;
- Fig. 5: eine weitere perspektivische Darstellung des Nadelmoduls aus Fig. 1 im Zustand ohne Last auf die Stecheinrichtung;
- Fig. 6: eine perspektivische Darstellung des Nadelmoduls aus Fig. 1 im Zustand mit Last auf die Stecheinrichtung;
- Fig. 7: eine weitere perspektivische Darstellung des Nadelmoduls aus Fig. 1 im Zustand mit Last auf die Stecheinrichtung bei härterer Dämpfungseinstellung;
- Fig. 8: eine perspektivische Darstellung eines anderen Nadelmoduls;
- Fig. 9: eine perspektivische Darstellung eines weiteren Nadelmoduls;
- Fig. 10: eine perspektivische Darstellung eines weiteren Nadelmoduls mit einstellbarem Dämpfungsgrad;
- Fig. 11: eine perspektivische Darstellung eines anderen Nadelmoduls mit einstellbarem Dämpfungsgrad und
- Fig. 12: eine Darstellung des anderen Nadelmoduls aus Fig. 11 im Schnitt.

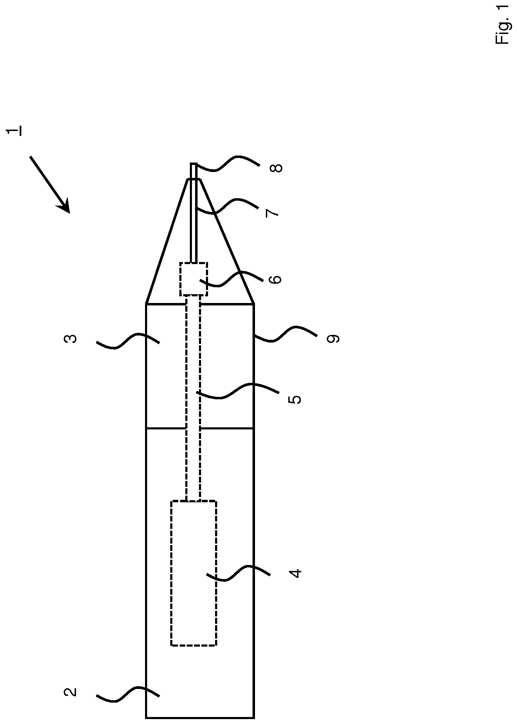

Fig. 1 zeigt eine schematische Darstellung eines Handgeräts 1 zum wiederholten Aufstechen einer menschlichen oder einer tierischen Haut. Das Handgerät 1 weist ein Antriebsmodul 2 sowie ein hieran koppelndes Nadelmodul 3 auf. In dem Antriebsmodul 2 ist eine Antriebseinrichtung 4 aufgenommen, beispielsweise ein elektrischer Motor, die eingerichtet ist, mit einer Wiederholfrequenz eine Antriebskraft oder -bewegung bereitzustellen, die über einen Kopplungsmechanismus 5 auf Funktionskomponenten 6 im Nadelmodul 3 übertragen wird.

Als Reaktion auf die angekoppelte Antriebsbewegung wird eine Stecheinrichtung 7 vor und zurück bewegt, sodass eine Stechspitze 8 der Stecheinrichtung 7 zumindest in einer distalen Stellung außerhalb eines Gehäuses 9 des Nadelmoduls 3 angeordnet ist (vgl. Fig. 1).

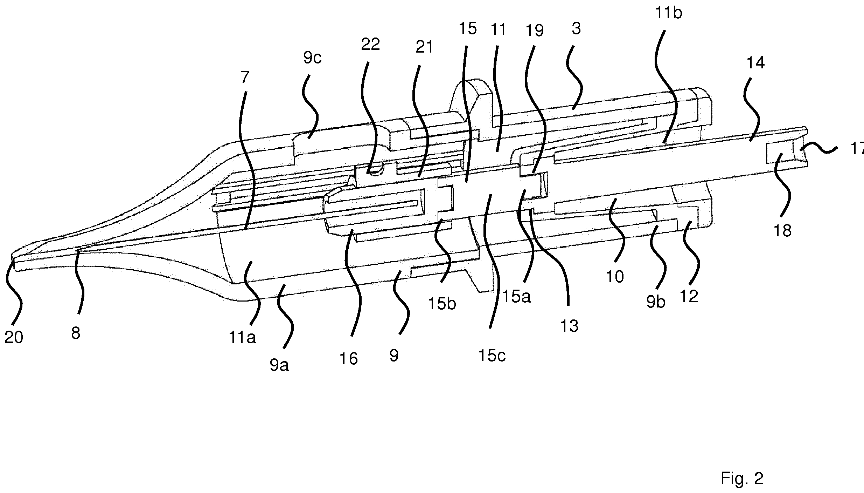

Im Folgenden werden unter Bezugnahme auf die Fig. 2 bis 10 Ausführungsformen für das Nadelmodul 3 weiter erläutert. Für gleiche Merkmale werden hierbei dieselben Bezugszeichen verwendet.

Fig. 2 zeigt eine perspektivische Darstellung einer Ausführung des Nadelmoduls 3 im Querschnitt. Das Gehäuse 9 weist ein vorderseitiges oder stechseitiges Gehäuseteil 9a sowie ein rückseitiges oder antriebsseitiges Gehäuseteil 9b auf, die lösbar oder nicht lösbar miteinander verbunden sind. Mithilfe einer elastischen Membran 10 ist ein Gehäuseinnenraum 11 in einen stechseitigen Gehäuseraum 11a sowie einen antriebsseitigen Gehäuseraum 11b unterteilt. Die elastische Membran 10 dichtet die beiden Gehäuseraum 11a, 11b gegeneinander ab, so dass ein Übertritt von Flüssigkeiten oder Substanzen aus dem stechseitigen Gehäuseraum 11a in den antriebsseitigen Gehäuseraum 11b verhindert ist. Bei der gezeigten Ausführungsform weist die elastische Membran 10 im Querschnitt eine Topfform auf und erstreckt sich abschnittsweise in Längsrichtung des Gehäuses 9. Die elastische Membran 10 ist an einem antriebsseitigen Ende 12 des Gehäuses 9 umlaufend dichtend festgelegt.

Bei der gezeigten Ausführungsform weist die elastische Membran 10 eine Membranöffnung 13 auf, an der rückseitig ein Koppelbauteil 14 angeordnet ist, wobei bei der gezeigten Ausführung beispielhaft eine formschlüssige Verbindung hergestellt ist. Das Koppelbauteil 14 dient dazu, eine von der Antriebseinrichtung 4 in dem Antriebsmodul 2 mit einer Wiederholfrequenz bereitgestellte Antriebsbewegung über ein Dämpfungsbauteil 15 auf eine Nadelaufnahme 16 einzuleiten, die in dem Gehäuse 9 in Längsrichtung verlagerbar angeordnet ist. Zum Koppeln an die Antriebseinrichtung 4 weist das Koppelbauteil 14 an einem antriebsseitigen Ende 17 eine Aufnahme 18 auf.

Stechseitig ist an dem Koppelbauteil 14 eine vordere Aufnahme 19 vorgesehen, in die ein antriebsseitiges Ende 15a des Dämpfungsbauteils 15 eingesteckt ist, beispielweise zum Ausbilden einer formschlüssigen Verbindung. Das antriebsseitige Ende 15a des Dämpfungsbauteils 15 durchgreift bei dem gezeigten Ausführungsbeispiel die Membranöffnung 13. Ein stechseitiges Ende 15b des Dämpfungsbauteils 15 lagert formschlüssig an der Nadelaufnahme 16.

An der Nadelaufnahme 16 ist die Stecheinrichtung 7 angeordnet, bei der es sich um eine Einzelnadel oder eine Nadelgruppe handeln kann.

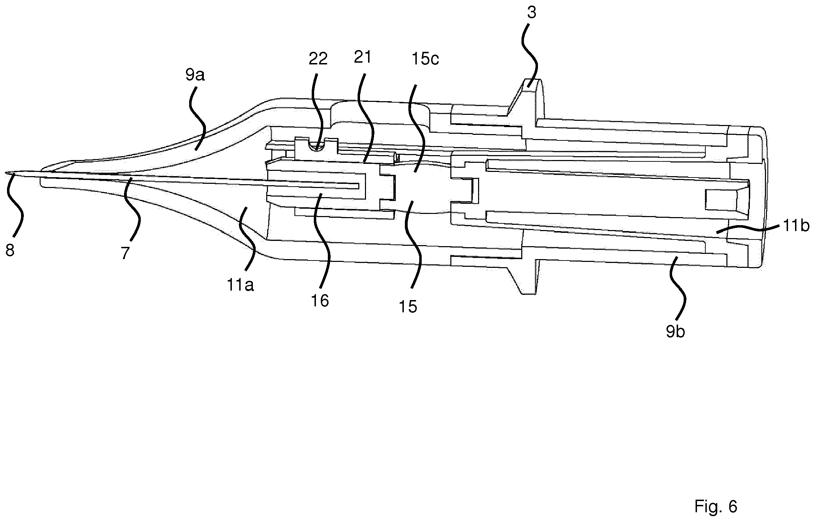

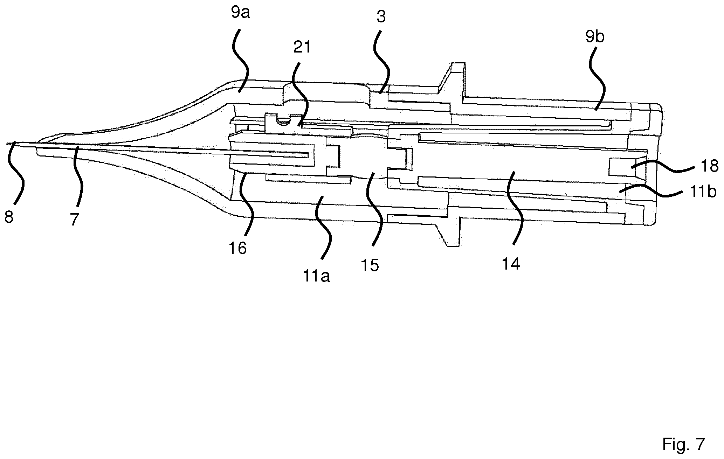

In einem mittleren Teil 15c kann das Dämpfungsbauteil 15 elastisch zusammengedrückt werden und hierbei seinen Durchmesser vergrößern (vgl. Fig. 6 und 7 unten).

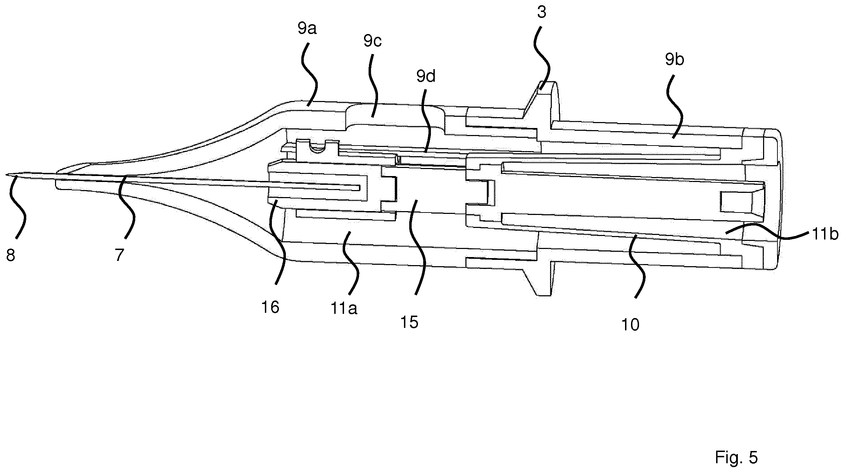

Infolge der auf die Nadelaufnahme 16 eingeleiteten Antriebsbewegung oder -kraft wird die Stecheinrichtung 7 durch eine vorderseitige Öffnung 20 am Gehäuse 9 vor und zurück bewegt, derart, dass die Nadelaufnahme 16 sowie die hieran angeordnete Stecheinrichtung 7 zwischen einer proximalen Stellung (vgl. Fig. 2) sowie einer distalen Stellung (vgl. Fig. 5 bis 7) verlagert werden, in welcher die Stechspitze 8 außerhalb des Gehäuses 8 und der vorderseitigen Öffnung 20 vorgelagert angeordnet ist.

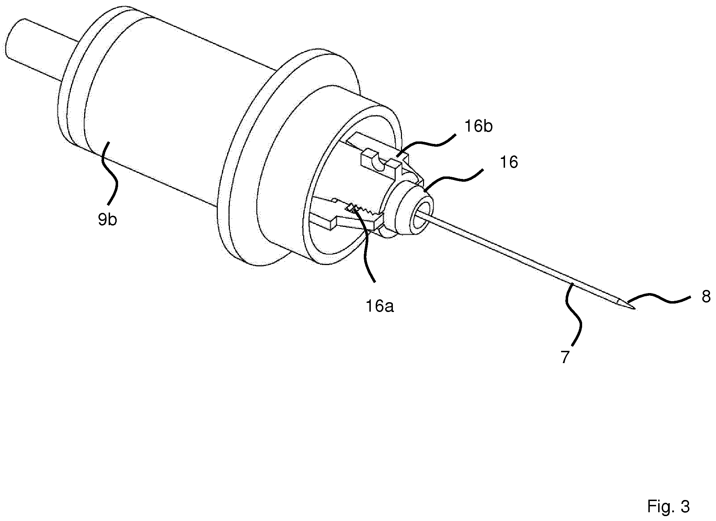

Fig. 3 zeigt eine perspektivische Darstellung von Elementen des Nadelmoduls aus Fig. 2 wobei der vordere und der hintere Gehäuseteil 9a, 9b getrennt sind.

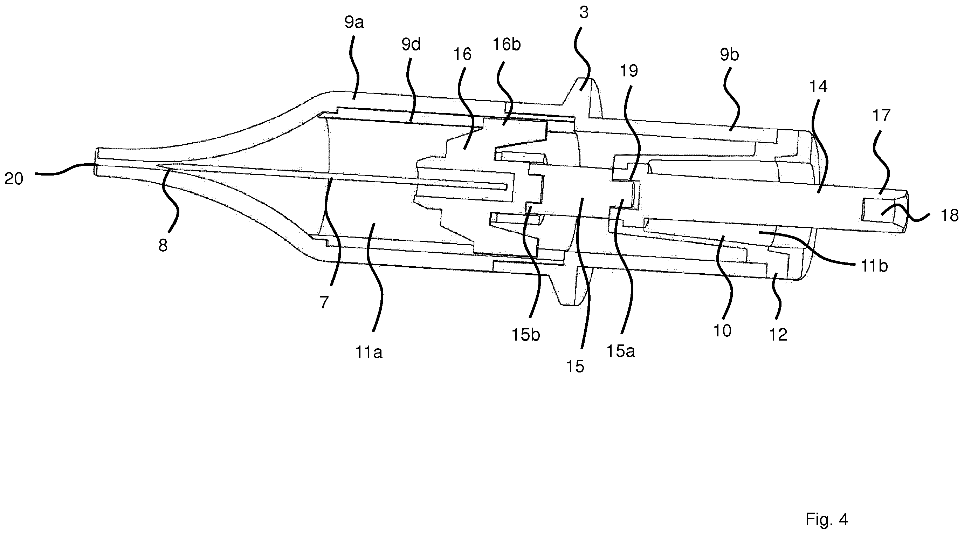

Die Fig. 4 bis 7 zeigen perspektivische Darstellungen des Nadelmoduls 2 aus Fig. 1. Die Darstellungen in Fig. 4 und 5 betreffen den Zustand, in welchem auf die Stecheinrichtung 7 keine Last wirkt. Fig. 6 zeigt den Zustand eines teilweise elastisch zusammengedrückten Dämpfungsbauteils 15, was die Folge eines Drucks oder einer Last auf die Stechspitze 8 ist, beispielsweise wenn diese auf die aufzustechende Haut trifft. Das Dämpfungsbauteil 15 entfaltet dann seine Dämpfungswirkung.

Bei der Darstellung in Fig. 7 wurde für das Dämpfungsbauteil 15 eine härtere Dämpfungseinstellung gewählt, indem ein Einstellbauteil 21, welches auf der Nadelaufnahme 16 verlagerbar angeordnet ist, in Richtung des Kopplungsbauteils 14 verschoben wurde, derart, dass eine Überlappung zwischen dem Einstellbauteil 21 und dem Dämpfungsbauteil 15 vergrößert ist. Hierdurch kann eine elastische Stauchung des Dämpfungsbauteils 15 begrenzt werden. Das Einstellbauteil 21 kann in den verschiedenen Verlagerungsstellungen gesichert sein, beispielweise mittels einer Rastverbindung 16a oder einer Schraube. Eine Ausführung mit einer verstellbaren Rastverbindung ist in Fig. 3 gezeigt.

Zum Verschieben weist das Einstellbauteil 21 ein Stellabschnitt 22 auf, an welchem zum Beispiel mit einem Schraubendreher angesetzt werden kann, um das Einstellelement 21 zu verlagern. Der Stellabschnitt 22 ist durch eine weitere Gehäuseöffnung 9c hindurch betätigbar.

Gemäß Fig. 5 ist am Gehäuse 9 innenseitig eine Führung 9d für die Nadelaufnahme 16 vorgesehen, wobei Flügel 16b entlang der Führung 9d geführt werden.

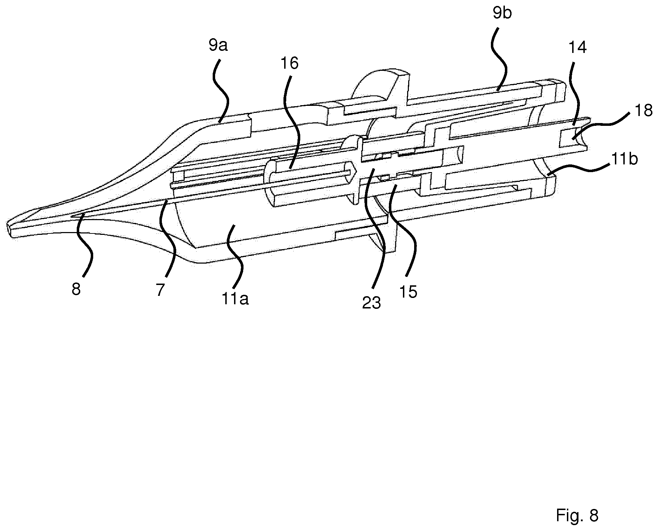

Fig. 8 zeigt eine perspektivische Darstellung eines weiteren Nadelmoduls 2, bei dem die Nadelaufnahme 16 und das Koppelbauteil 14 über eine Bauteilverlängerung 23 miteinander verbunden sind, die sich durch das Dämpfungsbauteil 15 hindurch erstreckt. Die Bauteilverlängerung 23 wird im Fall einer Last auf die Stechspitze 8 der Stecheinrichtung 7 verkürzt, wodurch sich Nadelaufnahme 16 und Koppelbauteil 14 annähern, wobei dieses durch das Dämpfungsbauteil 15 gedämpft wird.

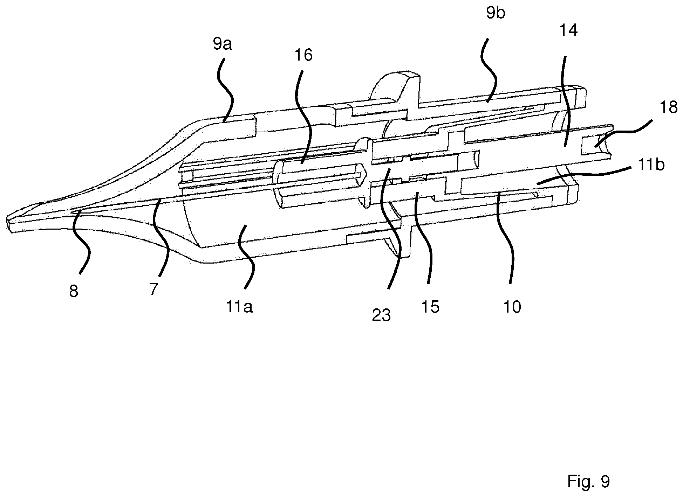

Bei der Ausführung in Fig. 9 ist das Dämpfungsbauteil 15 einstückig mit der Membran 10 gebildet. Das Dämpfungsbauteil 15 ist als eine vorderseitige Verlängerung der Membran 10 gebildet.

Auch bei den Ausführungsbeispielen aus den Fig. 8 und 9 kann eine Einstellbarkeit des Dämpfungsgrads vorgesehen sein, beispielweise vergleichbar dem Einstellbauteil 21.

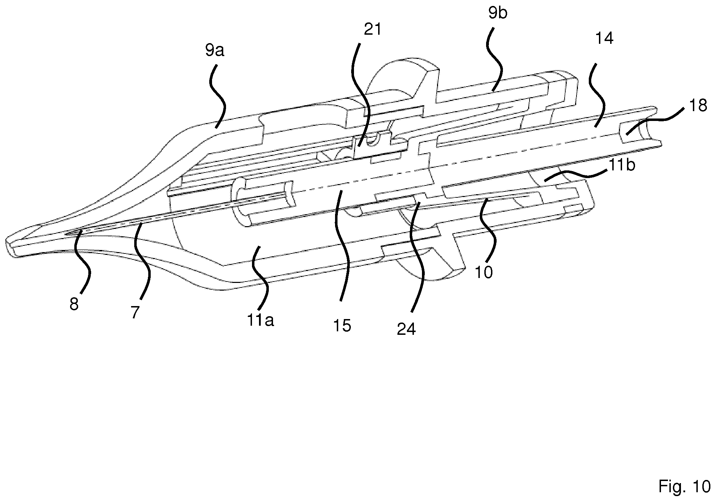

Fig. 10 zeigt eine perspektivische Darstellung eines weiteren Nadelmoduls 2 mit einstellbarem Dämpfungsgrad. Das Einstellbauteil 21 überlappt mit dem Dämpfungsbauteil 15, welches bei dieser Ausführung einstückig mit der Membran 10 ausgeführt und formschlüssig an das Koppelbauteil 14 angeformt ist, beispielsweise mittels 2K-Spritzgießen. Der vordere Bereich des Dämpfungsbauteils 15 weist eine Öffnung auf, in die die Stecheinrichtung 7 eingesetzt wird. Hierdurch entfällt die bei alternativen Ausführungen vorgesehene Nadelaufnahme 16. Diese Funktion wird von dem Dämpfungsbauteil 15 übernommen.

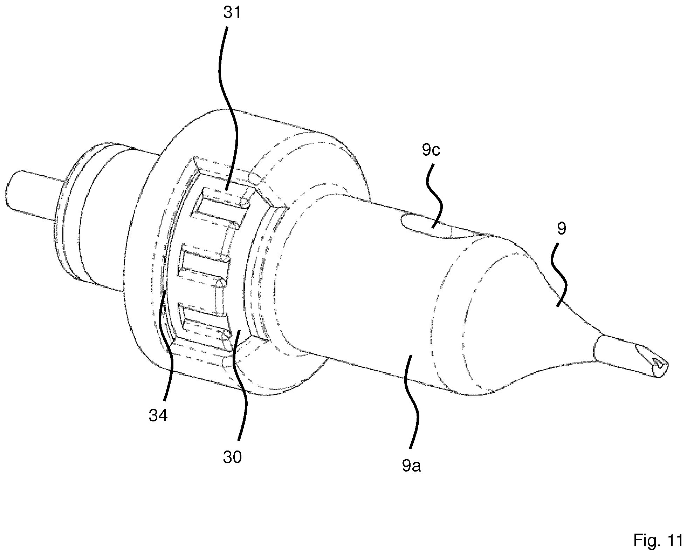

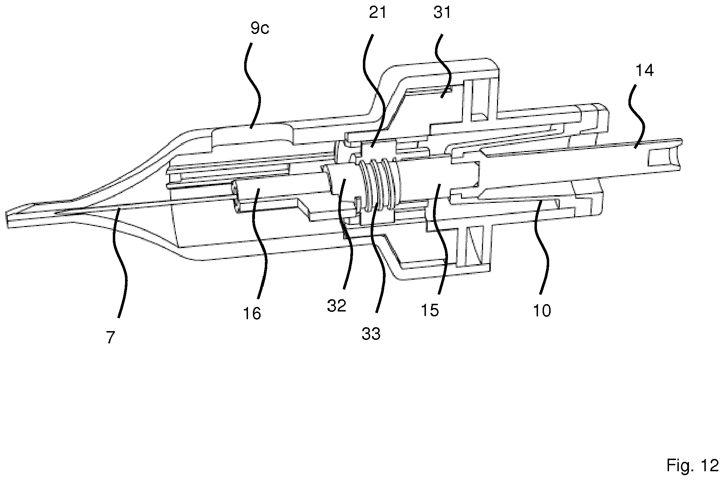

Fig. 11 und 12 zeigen eine perspektivische Darstellung sowie eine Schnittdarstellung eines anderen Nadelmoduls 2 mit einstellbarem Dämpfungsgrad. Es ist ein von außen zu betätigender Einstellmechanismus 30 vorgesehen, bei dem das Einstellbauteil 21 an eine von außen zugängliches Stellbauteil 31, welches als Stellrad ausgeführt ist, und ein im Gehäuse 9 aufgenommenes weiteres Stellbauteil 32 mit einem Gewinde 33 koppelt, derart, dass beim Drehen des Stellbauteils 31 das Einstellbauteil 21 mitgenommen und aufgrund seiner Kopplung an das weitere Stellbauteilauteil 32 mit dem Gewinde 33 in Längsrichtung verlagert wird, wodurch eine Überlappung mit dem Dämpfungsbauteil 15 eingestellt wird. Das Dämpfungsbauteil 15 koppelt hierbei rückseitig an das weitere Stellbauteil 32, welches vorderseitig an die Nadelaufnahme 16 koppelt.

Es kann vorgesehen sein, dass das weitere Stellbauteil 32 einstückig mit der Nadelaufnahme 16 gebildet ist, so dass in diesem Fall das Gewinde 33 an der Nadelaufnahme 16 angeordnet ist, an welche rückseitig das Dämpfungsbauteil 15 koppelt.

Das Stellbauteil 31 ist von außen über eine Gehäuseöffnung 34 betätigbar, insbesondere drehbar.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Nadelmodul für ein antriebsgesteuertes Handgerät zum wiederholten Aufstechen einer menschlichen oder einer tierischen Haut, mit
- einem Gehäuse (9);
- einer Nadelaufnahme (16), die in dem Gehäuse (9) in Längsrichtung des Gehäuse (9) vor und zurück verlagerbar angeordnet ist;
- einer Stecheinrichtung (7), die an der Nadelaufnahme (16) angeordnet ist, derart, dass die Stecheinrichtung (7) aufgrund der Bewegung der Nadelaufnahme (16) zwischen einer proximalen und einer distalen Stellung, in welcher eine Stechspitze (8) der Stecheinrichtung (7) außerhalb des Gehäuses (9) angeordnet ist, verlagerbar ist;
- einem Koppelbauteil (14), welches an die Nadelaufnahme (16) koppelt, derart, dass eine von einer an das Koppelbauteil (14) koppelbaren Antriebseinrichtung mit einer Wiederholfrequenz bereitgestellte Antriebsbewegung auf die Nadelaufnahme (16) einleitbar ist; und
- einem Dämpfungsbauteil (15), welches zwischen der Nadelaufnahme (16) und dem Koppelbauteil (14) angeordnet ist, so dass eine Kraftübertragung hierzwischen dämpfend ausführbar st.

2. Nadelmodul nach Anspruch 1, **dadurch gekennzeichnet, dass** das Dämpfungsbauteil (15) aus einem elastisch nachgebenden Material ist.

3. Nadelmodul nach Anspruch 1 oder 2, **dadurch gekennzeichnet, dass** gegenüberliegende Endabschnitte (15a, 15b) des Dämpfungsbauteils (15) jeweils direkt an die Nadelaufnahme (16) einerseits und das Koppelbauteil (14) andererseits koppeln.

4. Nadelmodul nach mindestens einem der vorangehenden Ansprüche, dadurch **gekennz**e**ichnet**, dass das Dämpfungsbauteils (15) an der Nadelaufnahme und / oder dem Koppelbauteil (14) gegen ein Verrutschen quer zur Längsrichtung des Gehäuses gesichert ist.

5. Nadelmodul nach mindestens einem der vorangehenden Ansprüche, **dadurch gekennzeichnet, dass** eine vom Dämpfungsbauteil (15) bereitgestellte Dämpfung mittels einer Einstelleinrichtung einstellbar ist.

6. Nadelmodul nach Anspruch 5, **dadurch gekennzeichnet, dass** die Einstelleinrichtung ein auf einer Innenwand des Gehäuses (9), an der Nadelaufnahme (16) und / oder an dem Koppelbauteil (14) verlagerbar angeordnetes Einstellbauteil (21) aufweist.

7. Nadelmodul nach Anspruch 5 oder 6, **dadurch gekennzeichnet, dass** die Einstelleinrichtung eingerichtet ist, einen Dämpfungsweg einzustellen.

8. Nadelmodul nach mindestens einem der Ansprüche 5 bis 7, **dadurch gekennzeichnet, dass** die Einstelleinrichtung von außerhalb des Gehäuses (9) bedienbar ist und einen Stellabschnitt (22) aufweist, welcher zum Bedienen von außen durch eine Substanzöffnung des Gehäuses (9) zugänglich ist.

9. Nadelmodul nach mindestens einem der vorangehenden Ansprüche, **dadurch gekennzeichnet, dass** die Nadelaufnahme (16) und das Koppelbauteil (14) über eine zugeordnete und eine Längsverkürzung aufgrund der Dämpfung des Dämpfungsbauteils (15) zulassende Bauteilverlängerung (23) miteinander verbunden sind.

10. Nadelmodul nach Anspruch 9, **dadurch gekennzeichnet, dass** sich die Bauteilverlängerung (23) längs des Dämpfungsbauteils (15) innerhalb und / oder außerhalb desselben erstreckt.

11. Nadelmodul nach mindestens einem der vorangehenden Ansprüche, **dadurch gekennzeichnet, dass** in dem Gehäuse (9) die Nadelaufnahme (16) und das Dämpfungsbauteil (15) in einem stechseitigen Gehäuseraum (11a) und das Kopplungsbauteil (14) in einem antriebsseitigen Gehäuseraum (11b) angeordnet sind, wobei der stechseitige und der antriebsseitige Gehäuseraum (11a, 11b) mittels einer Membran (10) getrennt sind.

12. Nadelmodul nach Anspruch 11, **dadurch gekennzeichnet, dass** das Dämpfungsbauteil (15) einstückig mit der Membran (10) ausgeführt ist.

13. Nadelmodul nach Anspruch 11 oder 12, **dadurch gekennzeichnet, dass** ein stechseitiges Ende des Koppelbauteils (14) an einer Membranöffnung angeordnet ist.

14. Nadelmodul nach mindestens einem der Ansprüche 11 bis 13, **dadurch gekennzeichnet, dass** die Membran (10) sich in Längsrichtung des Gehäuse (9) und das Koppelbauteil (14) umgreifend erstreckt.

15. Handgerät zum wiederholten Aufstechen einer menschlichen oder einer tierischen Haut, mit
- einem Antriebsmodul (2), welches eine Antriebseinrichtung (4) aufweist, mit der eine Antriebsbewegung mit einer Wiederholfrequenz bereitgestellt ist; und
- einem Nadelmodul (3) nach mindestens einem der vorangehenden Ansprüche, welches mit dem Antriebsmodul verbunden ist, derart, dass die Antriebsbewegung auf die Nadelaufnahme (16) mit der Stecheinrichtung (7) gekoppelt ist.

## Claims

1. A needle module for a drive-controlled handheld device for repeatedly piercing human or animal skin, comprising
- a housing (9);
- a needle receptacle (16) that is arranged in the housing (9) so as to be displaceable back and forth in the longitudinal direction of the housing (9);
- a piercing device (7) that is arranged on the needle receptacle (16) in such a way that, by virtue of the movement of the needle receptacle (16), the piercing device (7) can be displaced between a proximal and a distal position in which a piercing tip (8) of the piercing device (7) is arranged outside the housing (9);
- a coupling component (14) that couples to the needle receptacle (16) in such a way that a drive movement provided at a repetition frequency by a drive device that can be coupled to the coupling component (14) can be imparted to the needle receptacle (16); and
- a damping component (15) that is arranged between the needle receptacle (16) and the coupling component (14) so that a force can be transmitted therebetween in a damping manner.

2. The needle module according to claim 1, **characterized in that** the damping component (15) is made of an elastically resilient material.

3. The needle module according to claim 1 or 2, **characterized in that** opposite end portions (15a, 15b) of the damping component (15) couple directly to the needle receptacle (16) and to the coupling component (14), respectively.

4. The needle module according to at least one of the preceding claims, **characterized in that** the damping component (15) is secured on the needle receptacle and/or on the coupling component (14) against slipping transversely to the longitudinal direction of the housing.

5. The needle module according to at least one of the preceding claims, **characterized in that** damping provided by the damping component (15) can be adjusted by means of an adjusting device.

6. The needle module according to claim 5, **characterized in that** the adjusting device has an adjusting component (21) that is displaceably arranged on an inner wall of the housing (9), on the needle receptacle (16), and/or on the coupling component (14).

7. The needle module according to claim 5 or 6, **characterized in that** the adjusting device is configured to adjust a damping stroke.

8. The needle module according to at least one of claims 5 to 7, **characterized in that** the adjusting device can be operated from outside the housing (9) and has an adjusting portion (22) that can be accessed for operation from the outside through a substance opening in the housing (9).

9. The needle module according to at least one of the preceding claims, **characterized in that** the needle receptacle (16) and the coupling component (14) are interconnected by means of an associated component extension (23) that allows longitudinal shortening as a result of the damping of the damping component (15).

10. The needle module according to claim 9, **characterized in that** the component extension (23) extends along the damping component (15) inside and/or outside same.

11. The needle module according to at least one of the preceding claims, **characterized in that**, in the housing (9), the needle receptacle (16) and the damping component (15) are arranged in a piercing-side housing space (11a) and the coupling component (14) is arranged in a drive-side housing space (11b), the piercing-side and the drive-side housing spaces (11a, 11b) being separated by means of a membrane (10).

12. The needle module according to claim 11, **characterized in that** the damping component (15) is integrally formed with the membrane (10).

13. The needle module according to claim 11 or 12, **characterized in that** a piercing-side end of the coupling component (14) is arranged on a membrane opening.

14. The needle module according to at least one of claims 11 to 13, **characterized in that** the membrane (10) extends in the longitudinal direction of the housing (9) and encompasses the coupling component (14).

15. A handheld device for repeatedly piercing human or animal skin, comprising
- a drive module (2) having a drive device (4) by means of which a drive movement with a repetition frequency is provided; and
- a needle module (3) according to at least one of the preceding claims that is connected to the drive module such that the drive movement is coupled onto the needle receptacle (16) with the piercing device (7).

## Revendications

1. Module d'aiguilles pour un outil à main commandé par entraînement destiné au perçage répété d'une peau humaine ou animale, comportant
- un boîtier (9) ;
- un réceptacle d'aiguilles (16) qui est disposé dans le boîtier (9) de manière à pouvoir être déplacé d'avant en arrière dans la direction longitudinale du boîtier (9) ;
- un dispositif de perçage (7) disposé sur le réceptacle d'aiguilles (16) de telle manière que le dispositif de perçage (7) puisse être déplacé entre une position proximale et une position distale, dans lequel une pointe de perçage (8) du dispositif de perçage (7) est disposée à l'extérieur du boîtier (9), en raison du mouvement du réceptacle d'aiguilles (16) ;
- un composant d'accouplement (14) qui s'accouple au réceptacle d'aiguilles (16) de telle manière qu'un mouvement d'entraînement produit par un dispositif d'entraînement pouvant être accouplé au composant d'accouplement (14) avec une fréquence de répétition puisse être introduit dans le réceptacle d'aiguilles (16) ; et
- un composant d'amortissement (15) disposé entre le réceptacle d'aiguilles (16) et le composant d'accouplement (14), de sorte qu'une transmission de force entre eux puisse être effectuée de manière amortie.

2. Module d'aiguilles selon la revendication 1, **caractérisé en ce que** le composant d'amortissement (15) est constitué d'un matériau élastiquement déformable.

3. Module d'aiguilles selon la revendication 1 ou 2, **caractérisé en ce que** des sections d'extrémité opposées (15a, 15b) du composant d'amortissement (15) s'accouplent respectivement directement au récepteur d'aiguille (16), d'une part, et au composant d'accouplement (14), d'autre part.

4. Module d'aiguilles selon au moins l'une des revendications précédentes, **caractérisé en ce que** le composant d'amortissement (15) au niveau du réceptacle d'aiguilles et/ou du composant d'accouplement (14) est protégé contre le glissement de manière transversale à la direction longitudinale du boîtier.

5. Module d'aiguilles selon au moins l'une des revendications précédentes, **caractérisé en ce qu'**un amortissement produit par le composant d'amortissement (15) est réglable au moyen d'un dispositif de réglage.

6. Module d'aiguilles selon la revendication 5, **caractérisé en ce que** le dispositif de réglage comprend un composant de réglage (21) disposé de manière déplaçable sur une paroi intérieure du boîtier (9) au niveau du réceptacle d'aiguilles (16) et/ou du composant d'accouplement (14).

7. Module d'aiguilles selon la revendication 5 ou 6, **caractérisé en ce que** le dispositif de réglage est conçu pour régler une course d'amortissement.

8. Module d'aiguilles selon au moins l'une des revendications 5 à 7, **caractérisé en ce que** le dispositif de réglage peut être actionné de l'extérieur du boîtier (9) et comporte une section de réglage (22) qui est accessible pour l'actionnement de l'extérieur par une ouverture de substance du boîtier (9).

9. Module d'aiguilles selon au moins l'une des revendications précédentes, **caractérisé en ce que** le réceptacle d'aiguilles (16) et le composant d'accouplement (14) sont reliés par une extension de composant (23) associée qui permet un raccourcissement longitudinal en raison de l'amortissement du composant d'amortissement (15).

10. Module d'aiguilles selon la revendication 9, **caractérisé en ce que** l'extension de composant (23) s'étend le long du composant d'amortissement (15) à l'intérieur et/ou à l'extérieur de celui-ci.

11. Module d'aiguilles selon au moins l'une des revendications précédentes, **caractérisé en ce que** dans le boîtier (9), le réceptacle d'aiguilles (16) et le composant d'amortissement (15) sont disposés dans une chambre de boîtier (11a) du côté perçage et que le composant d'accouplement (14) est disposé dans une chambre de boîtier (11b) du côté entraînement, dans lequel les chambres de boîtier (11a, 11b) du côté perçage et du côté entraînement sont séparées au moyen d'une membrane (10).

12. Module d'aiguilles selon la revendication 11, **caractérisé en ce que** le composant d'amortissement (15) est réalisé d'une seule pièce avec la membrane (10).

13. Module d'aiguilles selon la revendication 11 ou 12, **caractérisé en ce qu'**une extrémité du côté perçage du composant d'accouplement (14) est disposée au niveau d'une ouverture de membrane.

14. Module d'aiguilles selon au moins l'une des revendications 11 à 13, **caractérisé en ce que** la membrane (10) s'étend dans la direction longitudinale du boîtier (9) et entoure le composant d'accouplement (14).

15. Outil à main destiné au perçage répété d'une peau humaine ou animale, comprenant
- un module d'entraînement (2) comportant un dispositif d'entraînement (4) produisant un mouvement d'entraînement présentant une fréquence de répétition ; et
- un module d'aiguilles (3) selon au moins l'une des revendications précédentes, qui est relié au module d'entraînement de telle manière que le mouvement d'entraînement au niveau du réceptacle d'aiguilles (16) soit accouplé au dispositif de perçage (7).
